# EUROPEAN PATENT APPLICATION

(11) **EP 1 489 073 A1**
(43) Date of publication of application: **22.12.2004**
(21) Application number: 03712733.9
(22) Date of filing: 18.03.2003
(51) Int. Cl.: C07D 277/46

(54) **PROCESS FORPREPARATION OF ALKOXYBENZAMIDES AND THIAZOLYL ISOCYANATES**

(30) Priority: 20.03.2002 JP 2002078524; 20.03.2002 JP 2002078528
(71) Applicant: MITSUI CHEMICALS, INC., Tokyo (JP)
(72) Inventor: AWANO, Hirokazu, c/o Mitsui Chemicals, Inc., Omuta-shi, Fukuoka 836-8610 (JP); KOITO, Mitsuo, c/o Mitsui Chemicals, Inc., Omuta-shi, Fukuoka 836-8610 (JP); KUSUMOTO, Masahiko, c/o Mitsui Chemicals, Inc., Minato-ku, Tokyo 1057117 (JP)
(74) Representative: Paget, Hugh Charles Edward
(86) International application number: PCT/JP2003/003211
(87) International publication number: WO 2003/078412

(57) **Abstract**

An alkoxybenzamide represented by the general formula (3): 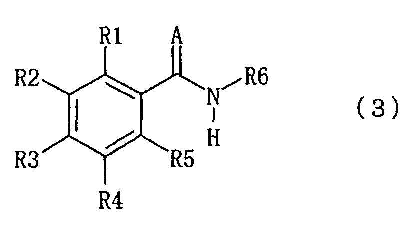 (wherein R1, R2, R3, R4, and R5 independently indicate a hydrogen atom, an alkyl group having one to six carbon atoms, or an alkoxy group having one to six carbon atoms; if R3 indicates a hydrogen atom or an alkyl group having one to six carbon atoms, R1 indicates an alkoxy group having one to six carbon atoms and R4 indicates an alkyl or alkoxy group having one to six carbon atoms; two adjacent substituents may be coupled with a cross-linking group to form a ring; R6 indicates a heteroaromatic ring; and A indicates an oxygen atom or a sulfur atom) is produced by reacting an alkoxybenzene represented by the general formula (1): 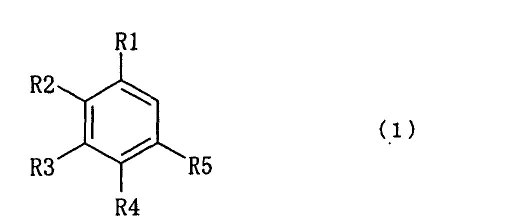 (wherein R1, R2, R3, R4, and R5 are defined above) with an isocyanate represented by the general formula (2):

R6―N=C=A (2)

(wherein R6 and A are defined above; and n indicates 1 or 2) in the presence of a Lewis acid. Among isocyanates represented by the general formula (2) is a thiazolyl isocyanate represented by the general formula (5): 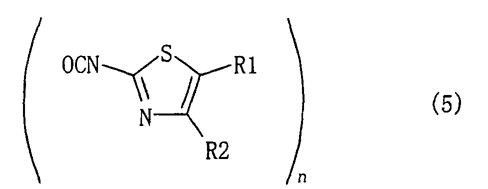 (wherein R1 indicates a hydrogen atom, a saturated or unsaturated alkyl group having one to six carbon atoms, a haloalkyl group having one to six carbon atoms, an alkoxy group having one to six carbon atoms, an alkoxycarbonyl group, or an acyl group; R2 indicates a hydrogen atom, a halogen atom, a saturated or unsaturated alkyl group having one to six carbon atoms, a haloalkyl group having one to six carbon atoms, an alkoxycarbonyl group having one to six carbon atoms, an acyl group, or a substituted or unsubstituted phenyl group; both R1 and R2 do not indicate a hydrogen atom; and n indicates 1 or 2). This thiazolyl isocyanate is produced from two precursors: an aminothiazole and a carbonyl halide. The present invention can provide a method for producing a high yield of alkoxybenzamide with fewer steps, and can also provide thiazolyl isocyanates, which are useful in the production of ureas, carbamates, and aromatic amides having a thiazole ring.

## Description

### Technical Field

The present invention relates to methods for producing alkoxybenzamides, which are useful as functional materials, such as medicines and agricultural chemicals, and as their intermediates. Specifically, the present invention relates to a method for producing trimethoxybenzoylaminothiazole derivatives, which have the effect of improving gastrointestinal motility. The present invention also relates to thiazolyl isocyanates, which are useful as starting materials for, for example, ureas, carbamates, or aromatic amides having a thiazole ring.

### Background Art

Alkoxybenzamides are useful as functional products, such as medicines and agricultural chemicals, and as their intermediates. Various methods for producing alkoxybenzamides have been studied.

In a general method (according to, for example, EP0870765), an alkoxybenzamide is produced by converting the corresponding alkoxybenzoic acid into its chloride or anhydride and reacting the chloride or anhydride with the corresponding amine.

The use of this method may be efficient if the alkoxybenzoic acid used as the starting material is a readily available material such as anisic acid. However, such alkoxybenzoic acids are limited and, even if available, are often very expensive. Thus, the alkoxybenzoic acid used must be produced. Alkoxybenzoic acids are produced by the following reported methods: 1) an alkoxybenzene is acylated into a ketone and is oxidized (for example, Tetrahedron. Lett. (41)3825(1971)); 2) an aldehyde group is introduced into an alkoxybenzene, which is then oxidized (for example, An. Quim. Ser. C 80(2)148(1984)); and 3) a hydroxyl group is introduced into a benzoic acid, which is then alkylated (for example, J. Org. Chem. 53(23)5519(1988)). However, these methods are unsatisfactory, often providing a low yield of alkoxybenzoic acid.

Methods for producing an alkoxybenzamide with an alkoxybenzoic acid, involving the amidation of the alkoxybenzoic acid, undesirably require many steps to produce the target compound.

Hence, a method including fewer steps has been desired for efficiently producing alkoxybenzamides.

Many ureas, carbamates, and aromatic amides having a thiazole ring are useful compounds having physiological activity. Examples of such known compounds include a 4-(pyridine-2-yl)-N-(4-phenylthiazole-2-yl)piperazine-1-carboxamide derivative (WO9300342), an N-phenyl-N'-(thiazole-2-yl)urea derivative (WO0026203) a 2-benzamide-N-thiazole-4-carboxamide derivative (U.S. Patent No. 5712270), a 2-(N-benzoylamino)-4-(aminocarbonyl)-1,3-thiazole derivative (EP0870765), a 4-guanidinobenzoate-4-[(N-thiazole-2-yl)carbamoyl]phenyl ester derivative (JP96048664), a 2-(3-benzylureido)thiazole-4-ylcarboxamide derivative (WO0024724), and an N-benzyl-N'-[4-(4-alkoxyphenyl)thiazole-2-yl]urea (WO9911637)

Carbamates are also useful as synthetic intermediates because the carbamate part of a carbamate can serve as a protective group. Examples of common protective groups for the amino group include a tert-butyloxycarbonyl group and a benzyloxycarbonyl group. An example of such carbamates is a 2-(2-aminothiazole-4-yl)acetic acid derivative (JP94087841), which has an amino group bonded to a carbamate protective group. This compound is useful as an intermediate of antibiotics.

According to a method reported in WO9300342, 4-pyridinyl-N-4-arylthiazolylpiperazine-1-carboxamides are produced from a 2-amino-4-arylthiazole, pyridinylpiperazine, and carbonyldiimidazole, among which the last one is very expensive. In addition, WO0026203 discloses a method for producing N-phenyl-N'-thiazolylurea from a phenyl isocyanate and an aminothiazole and a method for producing N-phenyl-N'-thiazolylurea from a thiazolyl isocyanate and an aniline derivative; however, only the former method has been put to practical use. Furthermore, according to methods disclosed in US5712270 and EP0870765, an N-thiazolylbenzamide is produced by converting the corresponding benzoic acid into an active species such as a chloride and reacting the active species with an aminothiazole. These methods, however, require two additional steps to provide the target compound.

Accordingly, an object of the present invention is to provide a method for producing a high yield of alkoxybenzamide with fewer steps. Another object of the present invention is to provide thiazolyl isocyanates, which are useful for producing ureas, carbamates, and aromatic amides having a thiazole ring.

The above examples of ureas, carbamates, and aromatic amides having a thiazole ring that have physiological activity include a thiazole skeleton introduced from an aminothiazole. The introduction of the thiazole skeleton may also be effectively performed using thiazolyl isocyanates; however, only a few thiazolyl isocyanates are known.

As a result of an intensive study for solving the above objects, the present inventors have found that alkoxybenzamides can be produced by reacting an alkoxybenzene with an isocyanate in the presence of a Lewis acid.

Furthermore, as a result of an intensive study on methods for producing thiazolyl isocyanates, which are useful for introducing a thiazole skeleton into a molecule, the present inventors have found that thiazolyl isocyanates can be readily produced by reacting an aminothiazole with a carbonyl halide. These findings led to the present invention.

### Disclosure of Invention

The present invention provides the following items:
(1) A method for producing an alkoxybenzamide represented by the general formula (3): 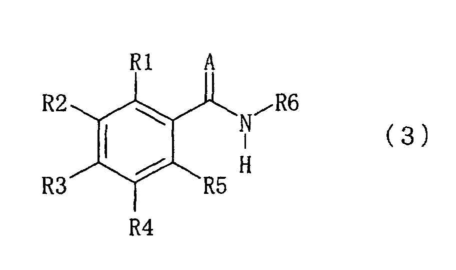 (wherein R1, R2, R3, R4, and R5 independently indicate a hydrogen atom, an alkyl group having one to six carbon atoms, or an alkoxy group having one to six carbon atoms; if R3 indicates a hydrogen atom or an alkyl group having one to six carbon atoms, R1 indicates an alkoxy group having one to six carbon atoms and R4 indicates an alkyl or alkoxy group having one to six carbon atoms; two adjacent substituents may be coupled with a cross-linking group to form a ring; R6 indicates a heteroaromatic ring; and A indicates an oxygen atom or a sulfur atom), the method including reacting an alkoxybenzene represented by the general formula (1): 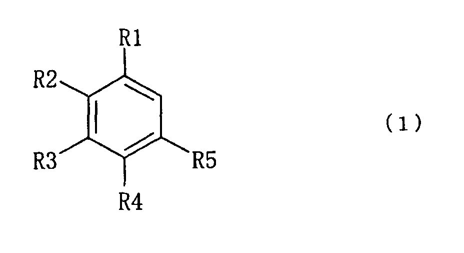 (wherein R1, R2, R3, R4, and R5 are defined above) with an isocyanate represented by the general formula (2):

   R6―N=C=A (2)

   (wherein R6 and A are defined above; and n indicates 1 or 2) in the presence of a Lewis acid;
(2) The method for producing the alkoxybenzamide according to Item (1), wherein, in the alkoxybenzene represented by the general formula (1) according to Item (1), R3 indicates an alkyl group having one to six carbon atoms or an alkoxyl group having one to six carbon atoms; R1, R2, R4, and R5 independently indicate a hydrogen atom, an alkyl group having one to six carbon atoms, or an alkoxy group having one to six carbon atoms; if R3 indicates an alkyl group having one to six carbon atoms, R1 indicates an alkoxy group having one to six carbon atoms; and two adjacent substituents may be coupled with a cross-linking group to form a ring;
(3) The method for producing the alkoxybenzamide according to Item (1), wherein, in the alkoxybenzene represented by the general formula (1) according to Item (1), R1, R3, and R4 independently indicate an alkoxy group;
(4) The method for producing the alkoxybenzamide according to any one of Items (1) to (3), wherein the isocyanate represented by the general formula (2) according to Item (1) is an isocyanate represented by the general formula (4): 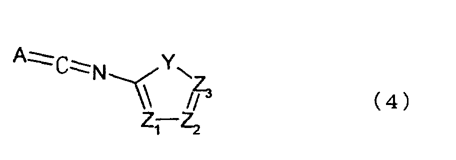 (wherein Y indicates an oxygen atom, a sulfur atom, or NR9; R9 indicates a saturated or unsaturated alkyl group or a substituted or unsubstituted phenyl group; Z₁, Z₂, and Z₃ independently indicate a nitrogen atom, CR7, or CR8; R7 and R8 independently indicate an hydrogen atom, a halogen atom, an alkyl group having one to six carbon atoms, a haloalkyl group having one to six carbon atoms, an alkoxyl group having one to six carbon atoms, an alkoxycarbonyl group, or a nitro group; and A is defined above);
(5) The method for producing the alkoxybenzamide according to Item (4), wherein, in the isocyanate represented by the general formula (4) according to Item (4), Y indicates an oxygen atom or a sulfur atom;
(6) The method for producing the alkoxybenzamide according to Item (5), wherein, in the isocyanate represented by the general formula (4) according to Item (4), Y indicates a sulfur atom; Z₁ indicates a nitrogen atom; and Z₂ and Z₃ independently indicate CR7 or CR8;
(7) The method for producing the alkoxybenzamide according to Item (1), wherein the alkoxybenzene represented by the general formula (1) according to Item (1) is 1,2,4-trimethoxybenzene; and the isocyanate represented by the general formula (2) according to Item (1) is a 4-(alkoxycarbonyl)thiazole-2-yl isocyanate;
(8) The method for producing the alkoxybenzamide according to any one of Items (1) to (7), wherein the Lewis acid contains tin ions;
(9) A thiazolyl isocyanate represented by the general formula (5): 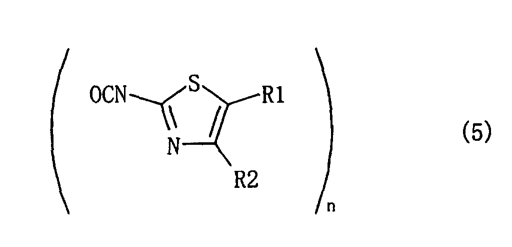 (wherein R1 indicates a hydrogen atom, a saturated or unsaturated alkyl group having one to six carbon atoms, a haloalkyl group having one to six carbon atoms, an alkoxy group having one to six carbon atoms, an alkoxycarbonyl group, or an acyl group; R2 indicates a hydrogen atom, a halogen atom, a saturated or unsaturated alkyl group having one to six carbon atoms, a haloalkyl group having one to six carbon atoms, an alkoxycarbonyl group having one to six carbon atoms, an acyl group, or a substituted or unsubstituted phenyl group; both R1 and R2 do not indicate a hydrogen atom; and n indicates 1 or 2);
(10) The thiazolyl isocyanate according to Item (9), wherein R1 indicates a hydrogen atom in the general formula (5); and
(11) The thiazolyl isocyanate according to Item (9) or (10), wherein R2 indicates an alkoxycarbonyl group having one to six carbon atoms in the general formula (5) according to Item (9).

### Best Mode for Carrying Out the Invention

In alkoxybenzenes represented by the general formula (1), R1, R2, R3, R4, and R5 independently indicate a hydrogen atom, an alkyl group having one to six carbon atoms, or an alkoxy group having one to six carbon atoms. Examples of the alkyl group having one to six carbon atoms include methyl, ethyl, n-propyl, isopropyl, n-butyl, isobutyl, sec-butyl, tert-butyl, and n-hexyl groups. Examples of the alkoxy group having one to six carbon atoms include methoxy, ethoxy, n-propoxy, isopropoxy, n-butoxy, isobutoxy, sec-butoxy, tert-butoxy, and n-hexyloxy groups. If R3 indicates a hydrogen atom or an alkyl group having one to six carbon atoms, R1 indicates an alkoxy group having one to six carbon atoms and R4 indicates an alkyl or alkoxy group having one to six carbon atoms. Two adjacent substituents may be coupled with a cross-linking group to form a ring. Alkoxybenzenes represented by the general formula (1) in which two adjacent substituents form a ring are exemplified by compounds having an aromatic ring condensed with a tetrahydropyran, dioxane, tetrahydrofuran, dioxolane, or benzene ring.

Examples of such alkoxybenzenes represented by the general formula (1) include 1,2-dimethoxybenzene, 1,3-dimethoxybenzene, 1,2,3-trimethoxybenzene, 1,2,4-trimethoxybenzene, 1,2,3,4-tetramethoxybenzene, 2-methoxytoluene, 3-methoxytoluene, 1,2-dimethyl-4-methoxybenzene, 1,3-dimethyl-2-methoxybenzene, 1,2-diethoxybenzene, 1,3-diethoxybenzene, 1,2,3-triethoxybenzene, 1,2,4-triethoxybenzene, 1,2-di-n-butoxybenzene, 1,3-di-n-butoxybenzene, 1,2,3-tri-n-butoxybenzene, 1,2,4-tri-n-butoxybenzene, chroman, 1,4-benzodioxane, 2,3-dihydrobenzofuran, and 1,2-methylenedioxybenzene.

In isocyanates represented by the general formula (2), R6 indicates a heteroaromatic ring such as pyridyl, pyranyl, pyrrolyl, furyl, thienyl, oxazolyl, isoxazolyl, thiazolyl, isothiazolyl, and 1,3,4-thiadiazolyl groups; A indicates an oxygen or sulfur atom; and n indicates 1 or 2.

Examples of such compounds represented by the general formula (2) include 2-pyridyl isocyanate, 2-pyranyl isocyanate, 2-pyrrolyl isocyanate, 2-furyl isocyanate, 2-thienyl isocyanate, 2-oxazolyl isocyanate, 5-isoxazolyl isocyanate, 2-thiazolyl isocyanate, 5-isothiazolyl isocyanate, 1,3,4-thiadiazole-2-isocyanate, 4-methyloxazole-2-isocyanate, 5-methyloxazole-2-isocyanate, 4-methylthiazole-2-isocyanate, 5-methylthiazole-2-isocyanate, 3-methylisoxazole-5-isocyanate, 3-methylisothiazole-5-isocyanate, 5-methyl-1,3,4-thiadiazole isocyanate, 4-chlorooxazole-2-isocyanate, 5-chlorooxazole-2-isocyanate, 4-chlorothiazole-2-isocyanate, 5-chlorothiazole-2-isocyanate, 3-chloroisoxazole-5-isocyanate, 3-chloroisothiazole-5-isocyanate, 5-chloro-1,3,4-thiadiazole isocyanate, 4-bromooxazole-2-isocyanate, 5-bromooxazole-2-isocyanate, 4-bromothiazole-2-isocyanate, 5-bromothiazole-2-isocyanate, 3-bromoisoxazole-5-isocyanate, 3-bromoisothiazole-5-isocyanate, 5-bromo-1,3,4-thiadiazole isocyanate, 4-chlorooxazole-2-isocyanate, 5-methoxycarbonyloxazole-2-isocyanate, 4-methoxycarbonylthiazole-2-isocyanate, 5-methoxycarbonylthiazole-2-isocyanate, 3-methoxycarbonyl isoxazole-5-isocyanate, 3-methoxycarbonylisothiazole-5-isocyanate, 5-methoxycarbonyl-1,3,4-thiadiazole isocyanate, 5-ethoxycarbonyloxazole-2-isocyanate, 4-ethoxycarbonylthiazole-2-isocyanate, 5-ethoxycarbonylthiazole-2-isocyanate, 3-ethoxycarbonylisoxazole-5-isocyanate, 3-ethoxycarbonylisothiazole-5-isocyanate, 5-ethoxycarbonyl-1,3,4-thiadiazole isocyanate, 2-pyridyl isothiocyanate, 2-pyranyl isothiocyanate, 2-pyrrolyl isothiocyanate, 2-furyl isothiocyanate, 2-thienyl isothiocyanate, 2-oxazolyl isothiocyanate, 5-isoxazolyl isothiocyanate, 2-thiazolyl isothiocyanate, 5-isothiazolyl isothiocyanate, 1,3,4-thiadiazole-2-isothiocyanate, 4-chloromethylthiazole-2-isocyanate, 5-chloromethylthiazole-2-isocyanate, and 5-t-butyl-1,3,4-thiadiazole-2-isocyanate.

Some isocyanates represented by the general formula (2) occur as a monomer, dimer, or mixture of a monomer and dimer. In the present invention, isocyanates represented by the general formula (2) include monomers, dimers, and mixtures of a monomer and dimer (the dimers will be exemplified later by thiazolyl isocyanates that belong to isocyanates represented by the general formula (2)).

An isocyanate represented by the general formula (2) may be synthesized by reacting the corresponding amine with a carbonyl halide, such as phosgene, or a thiocarbonyl halide, such as thiophosgene. Examples of the carbonyl halide used include phosgene, phosgene dimer, triphosgene, and chloroformates; among them, phosgene is economically preferred. Examples of the thiocarbonyl halide used include thiophosgene and chlorothioformates. The amount of carbonyl or thiocarbonyl halide used is at least equimolar to that of corresponding amine; preferably, the reaction solution is maintained at the saturated concentration to the completion of the reaction from the viewpoint of yields.

The reaction solvent used may be any solvent that is inert to the carbonyl halide, such as phosgene, and the isocyanate represented by the general formula (2). Examples of such a solvent include benzene; alkylbenzenes such as toluene, xylene, and propylbenzene; halobenzenes such as chlorobenzene, bromobenzene, dichlorobenzene, and dibromobenzene; nitrobenzene; halogenated hydrocarbons such as dichloromethane, chloroform, dichloroethane, and trichloroethane; and esters such as ethyl acetate, butyl acetate, amyl acetate, methyl propionate, and ethyl propionate.

The volume of solvent used is 3 to 50 times that of the amine, that is, the precursor of the isocyanate represented by the general formula (2).

After at least the equivalent amount of carbonyl halide is added at 40°C or lower, the temperature of the reaction solution is raised to keep the reaction at 50°C to 150°C. The content of carbonyl halide may decrease in the reaction system; preferably, the carbonyl halide is constantly supplied little by little to maintain the saturated concentration.

On the completion of the reaction, the reaction solution is degassed by blowing an inert gas such as nitrogen through the reaction solution. The resultant reaction mass is then cooled. If a crystal precipitates after the cooling, it may be filtered out to obtain the isocyanate represented by the general formula (2); otherwise, if a crystal does not precipitate, the solvent used may be removed to obtain the isocyanate represented by the general formula (2).

The process of producing an isocyanate represented by the general formula (4), which is an example of isocyanates represented by the general formula (2), involves the generation of an N-substituted carbamoyl halide represented by the general formula (6): 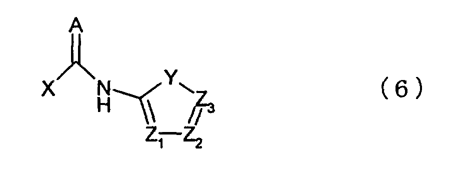 (where A, Y, Z₁, Z₂, and Z₃ are defined above; and X indicates a halogen atom). An isocyanate represented by the general formula (2), even if containing such an N-substituted carbamoyl halide, may be reacted with an alkoxybenzene represented by the general formula (1).

An alkoxybenzene represented by the general formula (1) and an isocyanate represented by the general formula (2) may be reacted in the presence of a Lewis acid to produce an alkoxyamide represented by the general formula (3).

The number of moles of the isocyanate represented by the general formula (2) is 0.1 to 10 times that of the alkoxybenzene. The isocyanate and the alkoxybenzene are used usually at a nearly equimolar ratio; if one of them is more expensive, the less expensive one is preferably oversupplied to consume the more expensive one completely. If the isocyanate used is a dimer, the amount of isocyanate used may be half. That is, the amount of dimer used is determined so as to provide a molar ratio as described above in terms of the monomer.

Examples of the Lewis acid used in this reaction include aluminum chloride, iron chloride(II), iron chloride(III), tin chloride(II), tin chloride(IV), and zinc chloride. If the isocyanate represented by the general formula (2) is a compound having a thiazole ring, the Lewis acid used is preferably tin chloride(IV). The Lewis acid may be used alone or in combination with another Lewis acid.

The reaction solvent used may be any solvent that is inert to the alkoxybenzene represented by the general formula (1) and the isocyanate represented by the general formula (2). Alternatively, the reaction solvent used may be any solvent if the alkoxybenzene used has sufficiently higher activity than the solvent. Examples of such a solvent include benzene; alkylbenzenes such as toluene, xylene, and ethylbenzene; halobenzenes such as chlorobenzene, dichlorobenzene, trichlorobenzene, and bromobenzene; nitrobenzene; halogenated saturated hydrocarbons; nitroalkanes such as nitromethane, nitroethane, and nitropropane; esters such as methyl acetate, ethyl acetate, butyl acetate, methyl propionate, ethyl propionate, and methyl benzoate; and alkyl ethers such as diethyl ether, diisopropyl ether, and dioxane. The solvent used may also be any mixture of the above solvents.

The volume of the solvent used is 3 to 50 times that of the alkoxybenzene represented by the general formula (1); preferably, the volume of the solvent used is 4 times or more for operationality and 20 times or less for economical efficiency.

This reaction is carried out within the range of -30°C to 150°C. If the isocyanate represented by the general formula (1) is a relatively less active isocyanate having a thiazole ring, the reaction is preferably carried out at 5°C or higher from the viewpoint of the rate of the reaction. An example of such an isocyanate is an isocyanate represented by the general formula (4), where Y indicates a sulfur atom, Z₁ indicates a nitrogen atom, and Z₂ and Z₃ independently indicate CR7 or CR8.

On the completion of the reaction, the alkoxybenzamide represented by the general formula (3) can be obtained by general processing; for example, an alcohol or water is added to the reaction solution, which is then stirred. If a crystal precipitates, the reaction solution may be filtered to obtain the target substance. If a sufficient amount of crystal does not precipitate, a crystal may be obtained by condensing the solvent used and adding another solvent to which the target substance is poorly soluble. The resultant compound may be directly used, or, if necessary, the purity of the compound may be increased, for example, by recrystallization or using a silica-gel column.

In thiazolyl isocyanates represented by the general formula (5), R1 indicates a hydrogen atom, a saturated or unsaturated alkyl group having one to six carbon atoms, a haloalkyl group having one to six carbon atoms, an alkoxy group having one to six carbon atoms, an alkoxycarbonyl group, or an acyl group. Examples of the saturated alkyl group having one to six carbon atoms include methyl, ethyl, n-propyl, isopropyl, n-butyl, isobutyl, sec-butyl, tert-butyl, and n-hexyl groups. Examples of the unsaturated alkyl group include allyl, 2-butenyl, and 3-butenyl groups. Examples of the haloalkyl group having one to six carbon atoms include chloromethyl, 2-chloroethyl, 2-bromoethyl, 1-chloroethyl, 3-chloropropyl, 3-bromopropyl, 2-chloropropyl, 1-chloropropyl, chloroisopropyl, 4-chloro-n-butyl, 4-bromo-n-butyl, 6-chloro-n-hexyl, and 6-bromo-n-hexyl groups. Examples of the alkoxy group having one to six carbon atoms include methoxy, ethoxy, n-propoxy, isopropoxy, n-butoxy, isobutoxy, sec-butoxy, tert-butoxy, n-hexyloxy, allyloxy, and 3-butenyloxy groups. Examples of the alkoxycarbonyl group include methoxycarbonyl, ethoxycarbonyl, n-propoxycarbonyl, isopropoxycarbonyl, n-butoxycarbonyl, and tert-butoxycarbonyl groups. Examples of the acyl group include acetyl, propionyl, butyryl, and hexanoyl groups. R2 indicates a hydrogen atom, a halogen atom, a saturated or unsaturated alkyl group having one to six carbon atoms, a haloalkyl group having one to six carbon atoms, an alkoxy group having one to six carbon atoms, an alkoxycarbonyl group, an acyl group, or a substituted or unsubstituted phenyl group. Examples of the halogen atom include fluorine, chlorine, bromine, and iodine atoms. Examples of the saturated alkyl group having one to six carbon atoms include methyl, ethyl, n-propyl, isopropyl, n-butyl, isobutyl, sec-butyl, tert-butyl, and n-hexyl groups. Examples of the unsaturated alkyl group include allyl, 2-butenyl, and 3-butenyl groups. Examples of the haloalkyl group having one to six carbon atoms include chloromethyl, 2-chloroethyl, 2-bromoethyl, 1-chloroethyl, 3-chloropropyl, 3-bromopropyl, 2-chloropropyl, 1-chloropropyl, chloroisopropyl, 4-chloro-n-butyl, 4-bromo-n-butyl, 6-chloro-n-hexyl, and 6-bromo-n-hexyl groups. Examples of the alkoxy group having one to six carbon atoms include methoxy, ethoxy, n-propoxy, isopropoxy, n-butoxy, isobutoxy, sec-butoxy, tert-butoxy, n-hexyloxy, allyloxy, and 3-butenyloxy groups. Examples of the alkoxycarbonyl group include methoxycarbonyl, ethoxycarbonyl, n-propoxycarbonyl, isopropoxycarbonyl, n-butoxycarbonyl, and tert-butoxycarbonyl groups. Examples of the acyl group include acetyl, propionyl, butyryl, and hexanoyl groups. Examples of the substituted or unsubstituted phenyl group include phenyl, tolyl, chlorophenyl, bromophenyl, methoxyphenyl, acetoxyphenyl, nitrophenyl, dimethylaminophenyl, dimethylphenyl, dichlorophenyl, and dibromophenyl groups. Both R1 and R2 do not indicate a hydrogen atom; and n indicates 1 or 2.

Examples of such thiazolyl isocyanates represented by the general formula (5) include 4-methylthiazole-2-yl isocyanate, 5-methylthiazole-2-yl isocyanate, 4,5-dimethylthiazole-2-yl isocyanate, 4-fluorothiazole-2-yl isocyanate, 4-chlorothiazole-2-yl isocyanate, 4-bromothiazole-2-yl isocyanate, 4-iodothiazole-2-yl isocyanate, 4-allylthiazole-2-yl isocyanate, 5-allylthiazole-2-yl isocyanate, 4-chloromethylthiazole-2-yl isocyanate, 5-chloromethylthiazole-2-yl isocyanate, 4-(2-chloroethyl)thiazole-2-yl isocyanate, 5-(2-chloroethyl)thiazole-2-yl isocyanate, 4-methoxythiazole-2-yl isocyanate, 5-methoxythiazole-2-yl isocyanate, 4-methoxycarbonylthiazole-2-yl isocyanate, 5-methoxycarbonylthiazole-2-yl isocyanate, 4-ethoxycarbonylthiazole-2-yl isocyanate, 5-ethoxycarbonyl-2-yl isocyanate, 4-acetylthiazole-2-yl isocyanate, 5-acetylthiazole-2-yl isocyanate, 4-benzoylthiazole-2-yl isocyanate, 5-benzoylthiazole-2-yl isocyanate, 4-phenylthiazole-2-yl isocyanate, 4-tolylthiazole-2-yl isocyanate, 4-chlorophenylthiazole-2-yl isocyanate, 4-methoxyphenylthiazole-2-yl isocyanate, and 4-(diacetoxyphenyl)thiazole-2-yl isocyanate.

Dimers of thiazolyl isocyanates represented by the general formula (5) in which n indicates 2 may have a structure represented by the formula (7): 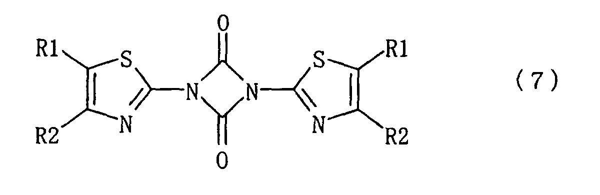 (where R1 and R2 are defined above); or may have a structure represented by the formula (8): 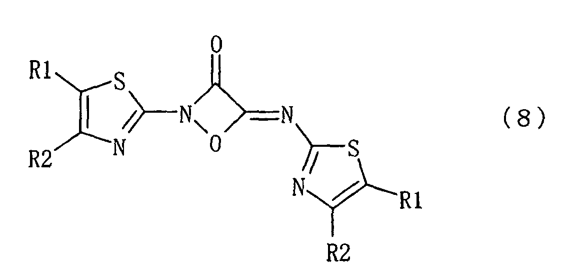 (where R1 and R2 are defined above). If these dimers are crystalline, they often have the structure represented by the general formula (7). On the other hand, if these dimers are dissolved in a solvent, they often have the structure represented by the general formula (8). These dimers may have either or both of these structures (7) and (8). The dimers, where n is 2, may be mixed with the monomers, where n is 1.

A thiazole isocyanate represented by the general formula (5) may be produced by suspending or dissolving an aminothiazole represented by the general formula (9): 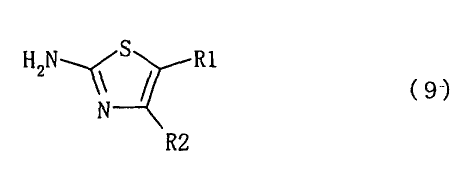 and/or its salt in a solvent and reacting the aminothiazole and/or its salt with a carbonyl halide usually in the atmosphere of an inert gas such as nitrogen, helium, and argon.

In the aminothiazole represented by the general formula (9), R1 and R2 are the same as those in the general formula (5) described above. Examples of the aminothiazole represented by the general formula (9) include 2-amino-4-methylthiazole, 2-amino-5-methylthiazole, 2-amino-4,5-dimethylthiazole, 2-amino-4-fluorothiazole, 2-amino-4-chlorothiazole, 2-amino-4-bromothiazole, 2-amino-4-iodothiazole, 2-amino-4-allylthiazole, 2-amino-5-allylthiazole, 2-amino-4-chloromethylthiazole, 2-amino-5-chloromethylthiazole, 2-amino-4-(2-chloroethyl)thiazole, 2-amino-5-(2-chloroethyl)thiazole, 2-amino-4-methoxythiazole, 2-amino-5-methoxythiazole, 2-amino-4-methoxycarbonylthiazole, 2-amino-5-methoxycarbonylthiazole, 2-amino-4-ethoxycarbonylthiazole, 2-amino-5-ethoxycarbonylthiazole, 2-amino-4-acetylthiazole, 2-amino-5-acetylthiazole, 2-amino-4-benzoylthiazole, 2-amino-5-benzoylthiazole, 2-amino-4-phenylthiazole, 2-amino-4-tolylthiazole, 2-amino-4-chlorophenylthiazole, 2-amino-4-methoxyphenylthiazole, and 2-amino-4-(diacetoxyphenyl)thiazole.

Examples of the salt of the aminothiazole represented by the general formula (9) include a hydrochloride, a bromate, a methanesulfonate, a toluenesulfonate, and an acetate. Such a salt may be prepared from the aminothiazole represented by the general formula (9) by a typical method. For example, the salt may be produced by dissolving or suspending the aminothiazole in any solvent and adding an equimolar amount of corresponding acid. If the salt precipitates, it may be directly filtered out; otherwise, if the salt remains dissolved, it may be obtained by removing the solvent used. If the solvent used is a protic solvent, such as an alcohol, or an amide, the resultant amine salt must be dried. If the solvent used is another type of solvent, either a salt containing the solvent or the solution or suspension containing the salt may be directly used in the reaction.

The reaction solvent used may be any solvent that is inert to the thiazole ring and substituents of the aminothiazole represented by the general formula (9), the carbonyl halide, and the product isocyanate. Examples of such a solvent include benzene; alkylbenzenes such as toluene, xylene, and propylbenzene; halobenzenes such as chlorobenzene, bromobenzene, dichlorobenzene, and dibromobenzene; nitrobenzene; halogenated hydrocarbons such as dichloromethane, chloroform, dichloroethane, and trichloroethane; and esters such as ethyl acetate, butyl acetate, amyl acetate, methyl propionate, and ethyl propionate.

The volume of solvent used is 3 to 50 times that of the aminothiazole.

Examples of the carbonyl halide used include phosgene, phosgene dimer, triphosgene, and chloroformates; among them, phosgene is economically preferred. The amount of carbonyl halide used is at least equimolar to that of the aminothiazole; preferably, the reaction solution is maintained at the saturated concentration to the completion of the reaction from the viewpoint of yields.

At least the equivalent amount of carbonyl halide is added to the reaction solution at 40°C or lower. The temperature of the reaction solution is then raised to keep the reaction at 50°C to 150°C. The content of carbonyl halide may decrease in the reaction system; preferably, the carbonyl halide is constantly supplied little by little to maintain the saturated concentration to the completion of the reaction.

The aminothiazole represented by the general formula (9) may be reacted with the carbonyl halide in the presence of an N,N-dialkylamide or a chain or cyclic urea to increase the rate of the reaction, thereby providing a higher yield. Examples of the N,N-dialkylamide used include N,N-dimethylformamide and N,N-dimethylacetoamide, while examples of the chain or cyclic urea used include tetramethylurea and N,N'-dimethylimidazolidinone. The amount of such a compound used is 0.1 to 10 mole percent of that of aminothiazole used.

On the completion of the reaction, usually, the thiazolyl isocyanate represented by the general formula (5) is recovered from the reaction solution after the removal of the remaining carbonyl halide. If the carbonyl halide used is phosgene, a nitrogen gas is blown through the reaction solution to remove the carbonyl halide. The reaction solution was then cooled. If a crystal precipitates after the cooling, it may be filtered out to obtain the thiazolyl isocyanate; otherwise, if a crystal does not precipitate, the solvent used may be removed to obtain the thiazolyl isocyanate.

If a salt of the aminothiazole represented by the general formula (9) is reacted with the carbonyl halide, the thiazolyl isocyanate represented by the general formula (5) may be obtained by the same process as for the aminothiazole. When a salt of the aminothiazole represented by the general formula (9) is used, the solution may be heated from the beginning of the addition of the carbonyl halide.

The resultant thiazolyl isocyanate represented by the general formula (5) may be directly used as the starting material for another reaction or, if necessary, may be refined by a common process such as recrystallization, reprecipitation, and distillation.

Thiazolyl isocyanates represented by the general formula (5) are useful as starting materials for ureas, carbamates, and aromatic amides: the reaction with an amine produces a thiazolylurea; the reaction with an alcohol produces a thiazolyl carbamate; and the reaction with an aromatic hydrocarbon in the presence of a Friedel-Crafts catalyst produces an N-thiazolylaromatic amide. These compounds are useful as functional products, such as medicines and agricultural chemicals, and their intermediates.

Examples of the present invention will now be described, though they do not limit the scope of the present invention.

### EXAMPLE 1

### Production of 4-ethoxycarbonylthiazole-2-yl isocyanate

First, 15 g of 2-amino-4-ethoxycarbonylthiazole was suspended in 120 mL of chlorobenzene. Then, 0.6 g of dimethylformamide was added to the reaction solution, through which 13 g of phosgene was blown at 15°C to 35°C. The temperature of the reaction solution was raised to 100°C, and the reaction solution was stirred for two hours. During the raising of the temperature, phosgene was kept blown through the reaction solution at about 4 g/hr. After the blowing of phosgene, a nitrogen gas was blown through the reaction solution for one hour. Then, the reaction solution was cooled. A precipitated crystal was filtered out and was washed with chlorobenzene to yield 16.9 g of the title compound, where the purity was 80%; and the yield converted according to the purity was 78%.

After 2 g of the resultant crystal was dissolved in 20 mL of ethanol, the solution was condensed to about 3 mL. A precipitated crystal was filtered out. As a result, 0.5 g of refined crystal was yielded.
The spectral data of the refined crystal is as follows:
1H-NMR(DMSO-d6) σ(ppm): 1.24(t, 3H), 1.30(t, 3H), 4.23(q, 2H), 4.31(q, 2H), 7.74(s, 1H), 8.69(s, 1H)
IR(KBr)(cm-1): 1770, 1740, 1701, 1593, 1560
FAB-MS: 397

### EXAMPLE 2

### Production of 4,5-dimethylthiazole-2-yl isocyanate

First, 10 g of 2-amino-4,5-dimethylthiazole was suspended in 100 mL of chlorobenzene. Then, 0.4 g of dimethylformamide was added to the reaction solution, through which 12 g of phosgene was blown at 15°C to 35°C. The temperature of the reaction solution was raised to 100°C, and the reaction solution was stirred for two hours. During the raising of the temperature, phosgene was kept blown through the reaction solution at about 4 g/hr. After the blowing of phosgene, a nitrogen gas was blown through the reaction solution for one hour. Then, the reaction solution was cooled. Since a tarry material was generated, a supernatant liquid was extracted by decantation and was concentrated in a vacuum. A precipitated crystal was filtered out and was washed with a small amount of hexane to yield 6.8 g of the title compound.
The spectral data is as follows:
1H-NMR(CDCl3) σ(ppm) : 2.23(s, 3H), 2.35(s, 3H) , 2.39 (s, 3H), 2.49(s, 3H)
EI-MS: 308(M+), 154

### EXAMPLE 3

Production of 4-(chloromethyl)thiazole-2-yl isocyanate

First, 10 g of 2-amino-4-(chloromethyl)thiazole was suspended in 100 mL of chlorobenzene. Then, 0.4 g of dimethylformamide was added to the reaction solution, through which 13 g of phosgene was blown at 15°C to 35°C. The temperature of the reaction solution was raised to 100°C, and the reaction solution was stirred for two hours. During the raising of the temperature, phosgene was kept blown through the reaction solution at about 4 g/hr. After the blowing of phosgene, a nitrogen gas was blown through the reaction solution for one hour. Then, the reaction solution was cooled. Since a tarry material was generated, a supernatant liquid was extracted by decantation and was concentrated in a vacuum. A precipitated crystal was filtered out and was washed with a small amount of hexane to yield 5.9 g of the title compound.
The spectral data is as follows:
1H-NMR(CDCl3) σ(ppm): 4.72(s, 2H), 4.88(s, 2H), 6.87(s, 1H), 7.55(s, 1H)
EI-MS: 348(M+), 174

### EXAMPLE 4

### Production of 2-[N-(2,4,5-trimethoxybenzoyl)amino]-4-ethoxycarbonyl-1,3-thiazole

First, 300 mg (1.5 mmol) of 4-ethoxycarbonyl-1,3-thiazole-2-isocyanate, 255 mg (1.5 mmol) of 1,2,4-trimethoxybenzene, and 3 mL of chlorobenzene were mixed. Then, 262 µL (2.3 mmol) of tin chloride (IV) was added to the mixture. The temperature of the mixture was raised to 100°C, and the mixture was stirred for three hours. The reaction solution was cooled, was mixed with 2 mL of ethanol, and was stirred. A precipitated crystal was filtered out and was washed with a small amount of ethanol to yield 388 mg (1.1 mmol) of the title compound, where the yield was 70%.
1H-NMR (DMSO-d6) σ (ppm) : 1.31(t, 3H), 3.77(s, 3H), 3.91(s, 3H), 4.01(s, 3H), 4.29(q, 2H), 6.85(s, 1H), 7.40(s, 1H), 8.09(s, 1H), 11.65(s, 1H)

### EXAMPLE 5

Production of 2-[N-(2,4,5-trimethoxybenzoyl)amino]-4,5-dimethyl-1,3-thiazole

First, 200 mg (1.3 mmol) of 4,5-dimethyl-1,3-thiazole-2-isocyanate, 218 mg (1.3 mmol) of 1,2,4-trimethoxybenzene, and 4 mL of chlorobenzene were mixed. Then, 228 µL (1.95 mmol) of tin chloride(IV) was added to the mixture. The temperature of the mixture was raised to 100°C, and the mixture was stirred for three hours. The reaction solution was cooled, was mixed with 2 mL of ethanol, and was stirred. The reaction solution was concentrated in a vacuum and was mixed with ethyl acetate. A precipitated crystal was filtered out and was washed with a small amount of ethyl acetate to yield 210 mg (0.65 mmol) of the title compound, where the yield was 50%.
1H-NMR(DMSO-d6) σ(ppm): 2.20(s, 3H), 2.28(s, 3H), 3.77(s, 3H), 3.91(s, 3H), 4.06(s, 3H), 6.86(s, 1H), 7.46(s, 1H), 11.28(s, 1H)

### EXAMPLE 6

### Production of 2-[N-(2,4-dimethoxybenzoyl)amino]-4,5-dimethyl-1,3-thiazole

First, 200 mg (1.3 mmol) of 4,5-dimethyl-1,3-thiazole-2-isocyanate, 179 mg (1.3 mmol) of 1,3-dimethoxybenzene, and 4 mL of chlorobenzene were mixed. Then, 228 µL (1.95 mmol) of tin chloride (IV) was added to the mixture. The temperature of the mixture was raised to 100°C, and the mixture was stirred for three hours. The reaction solution was cooled, was mixed with 2 mL of ethanol, and was stirred. The reaction solution was concentrated in a vacuum and was mixed with ethyl acetate. A precipitated crystal was filtered out and was washed with a small amount of ethyl acetate to yield 220 mg (0.75 mmol) of the title compound,
where the yield was 58%.
1H-NMR (DMSO-d6) σ(ppm): 2.14(s, 3H), 2.26(s, 3H), 3.85(s, 3H), 4.02(s, 3H), 6.70-6.73(m, 2H), 7.87(d, 1H), 11.32(s, 1H)

### EXAMPLE 7

### Production of 2-[N-(2,4,5-trimethoxybenzoyl)amino]-4-chloromethyl-1,3-thiazole

First, 200 mg (1.2 mmol) of 4-chloromethyl-1,3-thiazole-2-isocyanate, 193 mg (1.2 mmol) of 1,2,4-trimethoxybenzene, and 4 mL of chlorobenzene were mixed. Then, 201 µL (1.7 mmol) of tin chloride(IV) was added to the mixture. The temperature of the mixture was raised to 100°C, and the mixture was stirred for three hours. The reaction solution was cooled, was mixed with 2 mL of ethanol, and was stirred. The reaction solution was concentrated in a vacuum and was mixed with ethyl acetate. A precipitated crystal was filtered out and was washed with a small amount of ethyl acetate to yield 230 mg (0.71 mmol) of the title compound, where the yield was 59%.
1H-NMR(DMSO-d6) σ(ppm): 3.77(s, 3H), 3.91(s, 3H), 4.03(s, 3H), 4.74(s, 2H), 6.85(s, 1H), 7.31(s, 1H), 7.43(s, 1H), 11.43(s, 1H)

### EXAMPLE 8

### Production of 2-[N-(2,4-dimethoxybenzoyl)amino]-4-chloromethyl-1,3-thiazole

First, 200 mg (1.2 mmol) of 4-chloromethyl-1,3-thiazole-2-isocyanate, 158 mg (1.2 mmol) of 1,3-dimethoxybenzene, and 4 mL of chlorobenzene were mixed. Then, 201 µL (1.7 mmol) of tin chloride(IV) was added to the mixture. The temperature of the mixture was raised to 100°C, and the mixture was stirred for three hours. The reaction solution was cooled, was mixed with 2 mL of ethanol, and was stirred. The reaction solution was concentrated in a vacuum and was mixed with ethyl acetate. A precipitated crystal was filtered out and was washed with a small amount of ethyl acetate to yield 220 mg (0.70 mmol) of the title compound, where the yield was 61%.
1H-NMR(DMSO-d6) σ(ppm): 3.85(s, 3H), 3.95(s, 3H), 4.73(s, 2H), 6.61-6.72(m, 2H), 7.38(s, 1H), 7.80(d, 1H), 11.42(s, 1H)

### EXAMPLE 9

### Production of 2-[N-(2,4-dimethoxybenzoyl)amino]-5-t-butyl-1,3,4-thiadiazole

First, 200 mg (1.1 mmol) of 5-t-butyl-1,3,4-thiadiazole-2-isocyanate, 151 mg (1.1 mmol) of 1,3-dimethoxybenzene, and 4 mL of chlorobenzene were mixed. Then, 192 µL (1.6 mmol) of tin chloride(IV) was added to the mixture. The temperature of the mixture was raised to 100°C, and the mixture was stirred for three hours. The reaction solution was cooled, was mixed with 2 mL of ethanol, and was stirred. The reaction solution was concentrated in a vacuum and was mixed with ethyl acetate. A precipitated crystal was filtered out and was washed with a small amount of ethyl acetate to yield 120 mg (0.37 mmol) of the title compound, where the yield was 34%.
1H-NMR(DMSO-d6) σ(ppm): 1.41(s, 9H), 3.85(s, 3H), 3.95(s, 3H), 6.68-6.72(m, 2H), 7.75(d, 1H), 11.67(s, 1H)

### Industrial Applicability

The present invention can therefore provide a method for producing a high yield of alkoxybenzamide represented by the general formula (3) from an alkoxybenzene represented by the general formula (1) and an isocyanate represented by the general formula (2). The present invention can further provide thiazolyl isocyanates represented by the general formula (5), which are useful in the production of ureas, carbamates, and aromatic amides having a thiazole ring.

## Claims

1. A method for producing an alkoxybenzamide represented by the general formula (3): 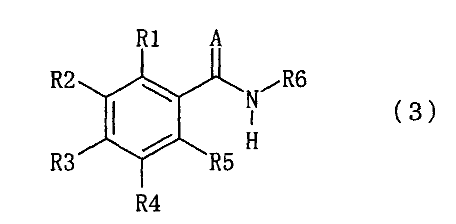 (wherein R1, R2, R3, R4, and R5 independently indicate a hydrogen atom, an alkyl group having one to six carbon atoms, or an alkoxy group having one to six carbon atoms; if R3 indicates a hydrogen atom or an alkyl group having one to six carbon atoms, R1 indicates an alkoxy group having one to six carbon atoms and R4 indicates an alkyl or alkoxy group having one to six carbon atoms; two adjacent substituents may be coupled with a cross-linking group to form a ring; R6 indicates a heteroaromatic ring; and A indicates an oxygen atom or a sulfur atom), the method comprising reacting an alkoxybenzene represented by the general formula (1): 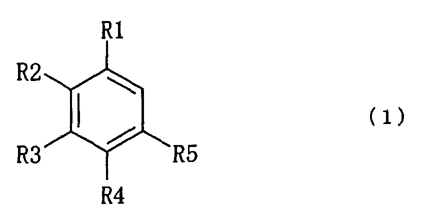 (wherein R1, R2, R3, R4, and R5 are defined above) with an isocyanate represented by the general formula (2) :
R6―N=C=A (2)
(wherein R6 and A are defined above; and n indicates 1 or 2) in the presence of a Lewis acid.

2. The method for producing the alkoxybenzamide according to Claim 1, wherein, in the alkoxybenzene represented by the general formula (1) according to Claim 1, R3 indicates an alkyl group having one to six carbon atoms or an alkoxyl group having one to six carbon atoms; R1, R2, R4, and R5 independently indicate a hydrogen atom, an alkyl group having one to six carbon atoms, or an alkoxy group having one to six carbon atoms; if R3 indicates an alkyl group having one to six carbon atoms, R1 indicates an alkoxy group having one to six carbon atoms; and two adjacent substituents may be coupled with a cross-linking group to form a ring.

3. The method for producing the alkoxybenzamide according to Claim 1, wherein, in the alkoxybenzene represented by the general formula (1) according to Claim 1, R1, R3, and R4 independently indicate an alkoxy group.

4. The method for producing the alkoxybenzamide according to any one of Claims 1 to 3, wherein the isocyanate represented by the general formula (2) according to Claim 1 is an isocyanate represented by the general formula (4): 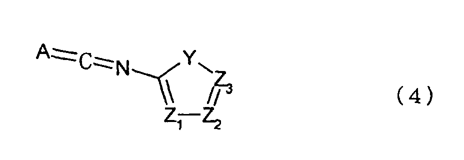 (wherein Y indicates an oxygen atom, a sulfur atom, or NR9; R9 indicates a saturated or unsaturated alkyl group or a substituted or unsubstituted phenyl group; Z₁, Z₂, and Z₃ independently indicate a nitrogen atom, CR7, or CR8; R7 and R8 independently indicate an hydrogen atom, a halogen atom, an alkyl group having one to six carbon atoms, a haloalkyl group having one to six carbon atoms, an alkoxyl group having one to six carbon atoms, an alkoxycarbonyl group, or a nitro group; A is defined above; and A is defined above).

5. The method for producing the alkoxybenzamide according to Claim 3, wherein, in the isocyanate represented by the general formula (4) according to Claim 4, Y indicates an oxygen atom or a sulfur atom.

6. The method for producing the alkoxybenzamide according to Claim 5, wherein, in the isocyanate represented by the general formula (4) according to Claim 4, Y indicates a sulfur atom; Z₁ indicates a nitrogen atom; and Z₂ and Z₃ independently indicate CR7 or CR8.

7. The method for producing the alkoxybenzamide according to Claim 1, wherein the alkoxybenzene represented by the general formula (1) according to Claim 1 is 1,2,4-trimethoxybenzene; and the isocyanate represented by the general formula (2) according to Claim 1 is a 4-(alkoxycarbonyl)thiazole-2-yl isocyanate.

8. The method for producing the alkoxybenzamide according to any one of Claims 1 to 7, wherein the Lewis acid contains tin ions.

9. A thiazolyl isocyanate represented by the general formula (5): 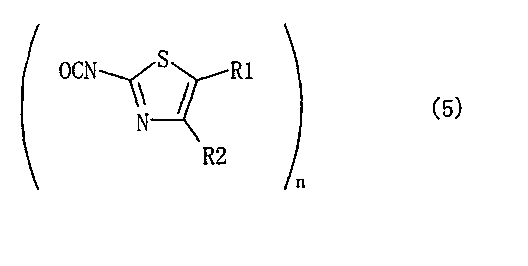 (wherein R1 indicates a hydrogen atom, a saturated or unsaturated alkyl group having one to six carbon atoms, a haloalkyl group having one to six carbon atoms, an alkoxy group having one to six carbon atoms, an alkoxycarbonyl group, or an acyl group; R2 indicates a hydrogen atom, a halogen atom, a saturated or unsaturated alkyl group having one to six carbon atoms, a haloalkyl group having one to six carbon atoms, an alkoxycarbonyl group having one to six carbon atoms, an acyl group, or a substituted or unsubstituted phenyl group; both R1 and R2 do not indicate a hydrogen atom; and n indicates 1 or 2).

10. The thiazolyl isocyanate according to Claim 9, wherein R1 indicates a hydrogen atom in the general formula (5).

11. The thiazolyl isocyanate according to Claim 9 or 10, wherein R2 indicates an alkoxycarbonyl group having one to six carbon atoms in the general formula (5) according to Claim 9.
